(19) Europäisches Patentamt
European Patent Office
Office européen des brevets

(11) Numéro de publication: **0 009 429**
**B1**

(12) **FASCICULE DE BREVET EUROPEEN**

(45) Date de publication du fascicule du brevet: **12.05.82**

(21) Numéro de dépôt: **79400603.1**

(22) Date de dépôt: **03.09.79**

(51) Int. Cl.³: **C 07 C 69/145,**
C 07 C 33/02,
C 07 C 43/15,
C 07 C 67/293,
C 07 C 29/46, C 07 C 41/30

(54) **Nouveaux composés undécadiènes fonctionnalisés comportant un substituant du type méthylène et leur procédé de fabrication.**

(30) Priorité: **15.09.78 FR 7826509**

(43) Date de publication de la demande:
**02.04.80 Bulletin 80/7**

(45) Mention de la délivrance du brevet:
**12.05.82 Bulletin 82/19**

(84) Etats contractants désignés:
**BE DE FR GB IT NL SE**

(56) Documents cités:
*néant*

(73) Titulaire: **RHONE-POULENC INDUSTRIES**
**22 Avenue Montaigne**
**F-75008 Paris (FR)**

(72) Inventeur: **Morel, Didier**
**19, avenue Jean Mermoz**
**F-69008 Lyon (FR)**

(74) Mandataire: **Chichery, Guy et al,**
**RHONE-POULENC RECHERCHES Centre de Recherches de Saint-Fons Service Brevets B.P. 62**
**F-69190 Saint-Fons (FR)**

**EP 0 009 429 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveaux composés undécadiènes fonctionnalisés comportant un substituant du type méthylène et leur procédé de fabrication

La présente invention a pour objet de nouveaux composés undécadiènes fonctionnalisés comportant un substituant du type méthylène. Au sens de la présente invention, on entend par substituant de type méthylène, le substituant méthylène lui-même $=CH_2$ et les substituants

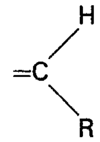 et 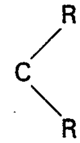

dans lesquels R représente un groupement alkyle.

L'invention concerne également un procédé de préparation de ces composés à partir de dimères fonctionnalisés de diènes conjugués en 1,3 et de diènes conjugués en 1,3.

On connait dans l'art antérieur un procédé d'addition de diènes conjugués en 1,3 et de dimères à chaine droite de butadiène.

C'est ainsi que le brevet français 71.02752 publié sous le numéro 2 077 072 décrit la réaction d'addition en présence d'un composé du rhodium entre des composés diènes conjugués en 1,3 de formule:

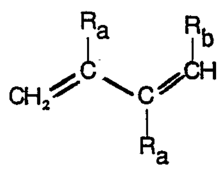 (A)

dans laquelle $R_a$ désigne un atome d'hydrogène ou un groupe alkyle et $R_b$ désigne un atome d'hydrogène, un groupe alkyle ou un groupe alkényle et des dérivés de dimères de butadiène, lesdits dimères ayant un groupe de substitution fixé en position 1 de formule:

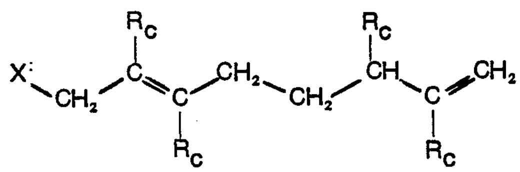 (B)

dans laquelle $R_c$ représente un atome d'hydrogène ou un groupe alkyle et X désigne un groupe hydroxy, alkoxy, aryloxy ou acyloxy.

Selon ce brevet, cette réaction produit une substance à chaine droite ayant une structure dans laquelle l'atome de carbone en position 8 de l'alcadiène substitué en 1 est lié à l'atome de carbone terminal de la chaine carbonée du composé diène conjugué en 1,3 et une substance ayant une structure dans laquelle l'atome de carbone en position 2 de l'alcadiène substitué en 1 est lié à l'atome de carbone terminal de la chaine carbonée du composé diène conjugué en 1,3.

Il est décrit à titre d'exemple que la réaction entre le butadiène-1,3

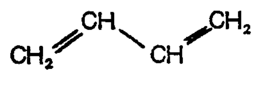 (1)

et l'acétoxy-1 octadiène-2,7

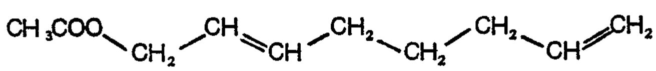 (2)

donne naissance au composé de type normal suivant (addition sur le carbone 8):

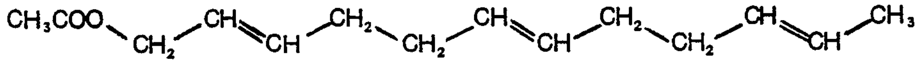 (3)

et aux composés de type iso suivants (addition sur le carbone 2):

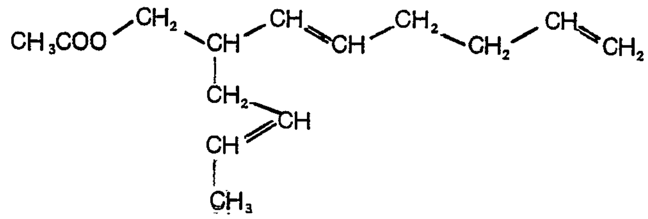
(4)

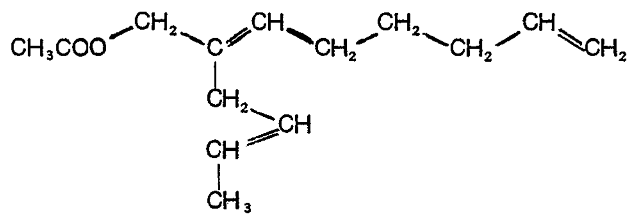
(5)

Il est également décrit dans ce même brevet de l'art antérieur que l'addition dans le milieu réactionnel d'un composé trivalent du phosphore tel que la triphenylphosphine, la tri-n-butylphosphine, la tri-isopropylphosphine, la tricyclohexylphosphine, la phenyldichlorophosphine, le trichlorure phosphoreux et la triphenylphosphite se traduit par une augmentation du composé de type normal sur le composé de type iso, c'est-à-dire favorise la production de composé de type normal. Selon les exemples, le rapport atomique du composé trivalent du phosphore au catalyseur au rhodium est inférieur ou approximativement égal à 1.

La demanderesse a découvert de façon surprenante que la mise en réaction des composés 1 et 2 en présence d'un catalyseur constitué d'un composé du rhodium et d'un composé trivalent du phosphore, ce dernier étant en quantité telle que le rapport atomique du phosphore trivalent au rhodium est supérieur ou égal à 1,5 donne naissance en tant que produit principal au composé de formule 6 ci-après:

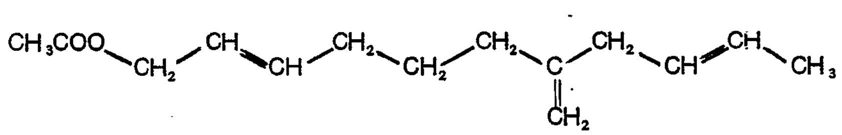
(6)

L'apparition, même en tant que produit secondaire, de ce composé de formule 6 n'est pas connue dans l'art antérieur. Ce composé 6 est prépondérant. Il y a bien entendu formation de composé 3, 4 et 5 en tant que produits secondaires.

D'une façon générale, la demanderesse a découvert également que cette réaction a lieu quand on met en réaction des composés du type A et des composés du type B en présence d'un système catalytique constitué d'un composé du rhodium et d'un composé trivalent du phosphore, le rapport atomique du phosphore trivalent au rhodium étant supérieur ou égal à 1,5.

L'invention a donc pour objet des composés undécadiènes fonctionnalisés comportant un substituant de type méthylène répondant à la formule générale:

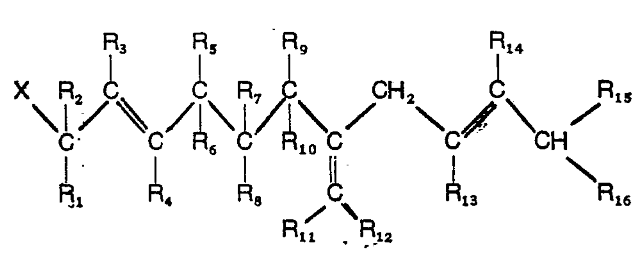
(I)

dans laquelle

X représente un groupement choisi parmi les groupements acyloxy, aryloxy, alkoxy et hydroxy.

$R_1$, $R_2$, $R_4$, $R_7$, $R_8$, $R_{13}$, $R_{14}$, $R_{15}$ et $R_{16}$ identiques ou différents représentent chacun un atome d'hydrogène ou un radical alkyle ayant de 1 à 3 atomes de carbone.

$R_3$, $R_5$ et $R_6$ représentent un atome d'hydrogène ou un radical alkyle ayant de 1 à 3 atomes de carbone, au moins l'un d'entre eux étant un atome d'hydrogène.

$R_9$, $R_{10}$, $R_{11}$ et $R_{12}$ représentent un atome d'hydrogène ou un radical alkyle ayant de 1 à 3 atomes de carbone, au moins l'un d'entre eux étant un atome d'hydrogène.

Préférentiellement, l'invention a pour objet des composés tels que définis ci-dessus dans lesquels:

$R_1$, $R_2$, $R_4$, $R_7$, $R_8$, $R_{13}$, $R_{14}$, $R_{15}$ et $R_{16}$ identiques ou différents représentent chacun un atome d'hydrogène ou un radical méthyle

$R_3$, $R_5$ et $R_6$ représentent un atome d'hydrogène ou un radical méthyle, au moins l'un d'entre eux étant un atome d'hydrogène

$R_9$, $R_{10}$, $R_{11}$ et $R_{12}$ représentent un atome d'hydrogène ou un radical méthyle, au moins l'un d'entre eux étant un atome d'hydrogène.

Encore plus préférentiellement, l'invention a pour objet les composés de formule (I) dans lesquels tous les R représentent des atomes d'hydrogène.

Parmi ceux-ci, l'invention vise en particulier, le composé suivant: acétoxy-1 méthylène-7 undécadiène 2,9 répondant à la formule:

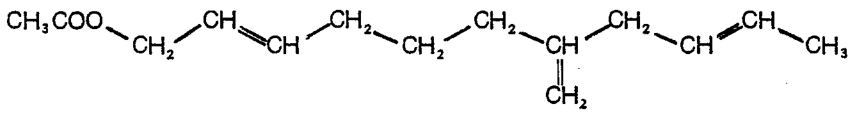

Un autre objet de l'invention est un procédé de préparation d'un composé de formule selon lequel on fait réagir:

— un dimère fonctionnalisé de diène conjugué de formule:

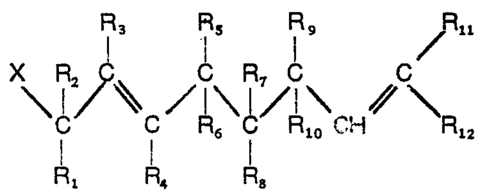 (II)

dans laquelle

X représente un groupement choisi parmi les groupements acyloxy, aryloxy, alkoxy et hydroxy

$R_1$, $R_2$, $R_4$, $R_7$ et $R_8$ identiques ou différents représentent chacun un atome d'hydrogène ou un radical alkyle ayant de 1 à 3 atomes de carbone

$R_3$, $R_5$ et $R_6$ représentent un atome d'hydrogène ou un radical alkyle ayant de 1 à 3 atomes de carbone, au moins l'un d'entre eux étant un atome d'hydrogène

$R_9$, $R_{10}$, $R_{11}$ et $R_{12}$ représentent un atome d'hydrogène ou un radical alkyle ayant de 1 à 3 atomes de carbone, au moins l'un d'entre eux étant un atome d'hydrogène.

— avec un diène conjugué de formule générale:

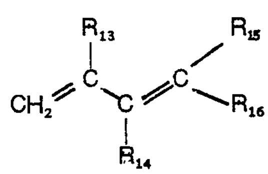 (III)

dans laquelle $R_{13}$, $R_{14}$, $R_{15}$ et $R_{16}$ identiques ou différents représentent chacun un atome d'hydrogène ou un radical alkyle ayant de 1 à 3 atomes de carbone

en présence d'un système catalytique constitué d'une part, par un composé du rhodium et d'autre part, par un composé trivalent du phosphore, le rapport atomique

$$\frac{\text{phosphore trivalent}}{\text{rhodium}}$$

étant supérieur ou égal à 1,5.

De préférence, le rapport atomique

$$\frac{\text{phosphore trivalent}}{\text{rhodium}}$$

est compris entre 1,5 et 10 et plus particulièrement entre 2 et 6.

Le composé du rhodium est choisi préférentiellement parmi les sels d'acides inorganiques du rhodium comme par exemple le Rh $Cl_3$.

Le composé trivalent du phosphore est choisi préférentiellement parmi les aryl et alkyl phosphines, comme par exemple la triphénylphosphine et la bis (diphenylphosphino)-1,2 éthylène de formule:

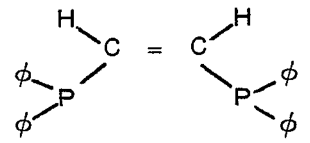

Selon un mode de réalisation particulier, on utilise simultanément la triphénylphosphine et Rh $Cl_3$.

L'invention concerne plus particulièrement un procédé tel que défini ci-dessus, selon lequel on utilise le composé de formule (II) dans laquelle

$R_1$, $R_2$, $R_4$, $R_7$ et $R_8$ identiques ou différents représentent chacun un atome d'hydrogène ou un radical méthyle

$R_3$, $R_5$ et $R_6$ représentent un atome d'hydrogène ou un radical méthyle, au moins l'un d'entre eux étant un atome d'hydrogène

$R_9$, $R_{10}$, $R_{11}$ et $R_{12}$ représentent un atome d'hydrogène ou un radical méthyle, au moins l'un d'entre eux étant un atome d'hydrogène.

On utilise de même, plus particulièrement, un composé de formule III dans laquelle $R_{13}$, $R_{14}$, $R_{15}$ et $R_{16}$ identiques ou différents représentent chacun un atome d'hydrogène ou un radical méthyle.

Selon un mode de réalisation particulier de l'invention, on fait réagir l'acétoxy-1 octadiène-2,7 de formule:

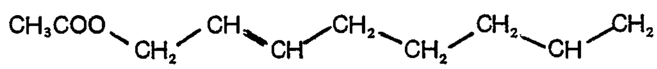

avec le butadiène-1,3 de formule:

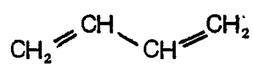

pour obtenir l'acétoxy-1 méthylène-7 undécadiène-2,9.

De même, la réaction selon l'invention de l'octadiène-2,7 ol-1 avec le butadiène-1,3 donne le méthylène-7 undécadiène-2,9 ol-1. La réaction du méthoxy-1 octadiène-2,7 avec le butadiène-1,3 donne le méthoxy-1 méthylène-7 undécadiène-2,9. Comme exemples de composés de formule (II), on peut encore citer l'acétoxy-1 diméthyl-2,6 octadiène-2,7, le méthoxy-1 dimethyl-2,6 octadiène-2,7, l'acétoxy-2 décadiène-3,8, le méthoxy-2 décadiène-3,8 et le décadiène-3,8 ol-2. Comme autres exemples de formule (III), on peut citer l'isoprène et le piperylène.

Le procédé selon l'invention est mis en oeuvre à une température généralement comprise entre 90°C et 150°C.

On opère généralement de la façon suivante: on utilise un autoclave en acier inoxydable dans lequel on introduit, sous atmosphère inerte, les réactifs préférentiellement dans l'ordre suivant: composé du rhodium, composé trivalent du phosphore, éthanol, dimère fonctionnalisé du diène conjugué et diène conjugué.

L'éthanol qui est utilisé en faible quantité permet l'activation du système catalytique. On chauffe sous agitation. Lorsque la réaction est terminée, on élimine le butadiène résiduel par dégazage. Le produit de la réaction est ensuite isolé du mélange obtenu par distillation sous pression réduite.

Le produit obtenu répondant à la formule I est utilisé après hydrogénation et saponification, comme composé intermédiaire pour la préparation d'alcools précurseurs de composés utilisables en tant que détergents biodégradables.

Les dimères de diènes fonctionnalisés de formule II sont préparés selon les techniques de l'art antérieur. On peut en particulier les préparer en faisant réagir des diènes conjugués avec des composés contenant des hydrogène actifs en présence de catalyseurs au palladium.

L'invention va maintenant être plus complètement décrite, à l'aide des exemples suivants qui ne sauraient en aucune manière être considérés comme une limitation de l'invention.

Exemple 1

Préparation de l'acétoxy-1 méthylène-7 undécadiène 2,9 à partir de butadiène-1,3 et d'acétoxy-1 octadiène-2,7.

$$\left(\text{Rapport atomique } \frac{\text{phosphore trivalent}}{\text{rhodium}} = 3\right)$$

On introduit dans un autoclave en acier inoxydable de 125 cc, 0,112 g de Rh $Cl_3$, $4H_2O$ (0,4 millimole), 0,314 g de triphénylphosphine (1,2 millimole); 2,60 g d'éthanol, 20 g d'acétoxy-1 octadiène

2,7 et 11 g de butadiène. On chauffe sous agitation à 120°C pendant 6 heures et demie. On élimine le butadiène n'ayant pas réagi et on obtient 32,4 g de mélange réactionnel rouge homogène dont l'analyse chromatographique en phase gazeuse et l'analyse infrarouge, RMN$^1$H et spectrographique de masse montrent qu'il est constitué de 0,82 g de composé iso de formule:

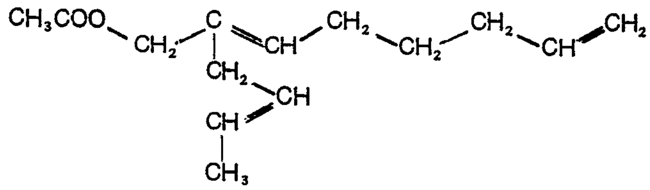

1,12 g de composé linéaire de formule:

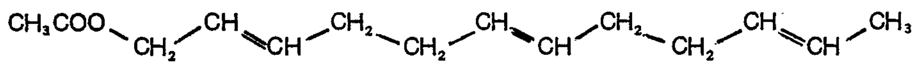

et 10,06 g du composé selon l'invention de formule (Eb = 80 – 84°C sous 0,2 mm de Hg)

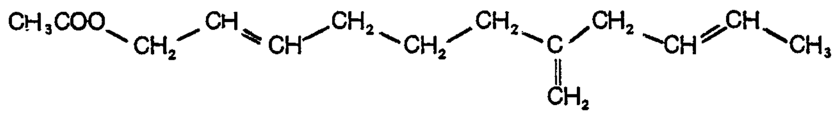

ainsi que 5 g d'acétoxy-1 octadiène 2,7 n'ayant pas réagi, 2,2 g d'alcool obtenu par réaction de trans-estérification entre les acétoxy et l'éthanol, le reste étant constitué principalement d'oligomères du butadiène.

La conversion de l'acétoxy-1 octadiène-2,7 est de 75%. La sélectivité en acétoxy-1 méthylène-7 undécadiène-2,7 est de 84%.

Exemple 2

Préparation de l'acétoxy-1 méthylène-7 undécadiène-2,9 à partir de butadiène-1,3 et d'acétoxy-1 octadiène-2,7

$$\text{(rapport atomique } \frac{\text{phosphore trivalent}}{\text{rhodium}} = 2\text{).}$$

On opère comme dans l'exemple 1 mais on utilise 0,210 g de triphénylphosphine (0,8 millimole).

On obtient:

— 0,72 g de composé iso

— 2,10 g de composé linéaire

— 7,67 g de composé selon l'invention

La sélectivité en produit selon l'invention est de 73% pour un taux de transformation de l'acétoxy-1 octadiène-2,7 de 79%.

Exemple 3: (Exemple comparatif)

Préparation de l'acétoxy-1-méthylène-7 undécadiène-2,9 à partir de butadiène-1,3 et d'acétoxy-1 octadiène-2,7.

$$\text{(Rapport atomique } \frac{\text{phosphore trivalent}}{\text{rhodium}} = 1\text{)}$$

On opère exactement dans les mêmes conditions que celles de l'exemple 1 mais on utilise seulement 0,105 g de triphénylphosphine (0,4 millimole).

On obtient 33,1 g de mélange réactionnel composé de:

— 3,06 g de composé iso

— 6,6 g de composé linéaire

— 1,6 g d'acétoxy-1 méthylène-7 undécadiène-2,9.

La sélectivité en acétoxy-1 méthylène-7 undécadiène-2,9 est de 15% et la conversion de l'acétoxy-1 octadiène-2,7 est de 77%.

Cet exemple montre clairement l'influence du rapport

$$\frac{\text{phosphore trivalent}}{\text{rhodium}}$$

quant à la formation du composé selon l'invention.

Exemple 4

Préparation de l'acétoxy-1 méthylène-7 undécadiène-2,9 à partir de butadiène-1,3 et d'acétoxy-1 octadiène-2,7

$$\text{(rapport atomique } \frac{\text{phosphore trivalent}}{\text{rhodium}} = 3).$$

On opère comme dans l'exemple 1 mais on utilise: 0,056 g de Rh $Cl_3$, $4H_2O$ (0,2 millimole), 0,157 g de triphénylphosphine (0,6 millimole) et 1 g d'éthanol.

On obtient:

— 0,3 g du composé iso
— 1,2 g du composé linéaire
— 9,8 g d'acétoxy-1 méthylène-7 undécadiène-2,7.

La sélectivité en acétoxy-1 méthylène-7 undécadiène-2,7 est de 86%.

Le taux de transformation de l'acétoxy-1 octadiène 2,7 est de 64%.

La quantité de Rh $Cl_3$, $4H_2O$ utilisée étant, dans cet exemple, plus faible, on obtient beaucoup moins de produits lourds pour un taux de transformation qui n'a pas diminué dans le même rapport que la quantité de rhodium.

Exemple 5

Préparation de méthylène-7 undécadiène-2,9 ol-1 à partir d'octadiène-2,7 ol-1 et de butadiène

$$\text{(rapport atomique } \frac{\text{phosphore trivalent}}{\text{rhodium}} = 3).$$

On introduit dans un autoclave en acier inoxydable de 125 cc, 0,168 g de Rh Cl3, $4H_2O$ (0,6 millimole), 0,476 g de triphénylphosphine (1,8 millimole), 0,8 g d'éthanol, 22,7 g d'octadiène-2,7 ol-1 et 9,8 g de butadiène 1,3. On chauffe sous agitation à 120°C pendant 6 heures. On élimine le butadiène n'ayant pas réagi.

On obtient 29,7 g d'un liquide orangé comportant quelques particules insolubles.

L'analyse chromatographique en phase gazeuse et l'analyse I,R RMN et spectrographique de masse montrent que le mélange réactionnel obtenu est constitué par:

. 2,7 g de produit iso de formule:

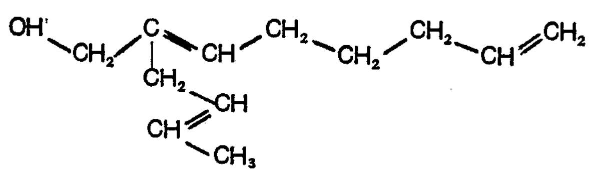

. 0,87 g de produit lineaire de formule:

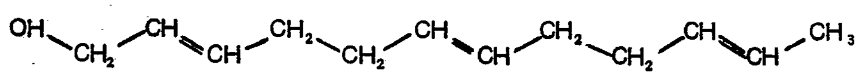

. 7 g de méthylène-7 undécadiène 2,9 ol-1

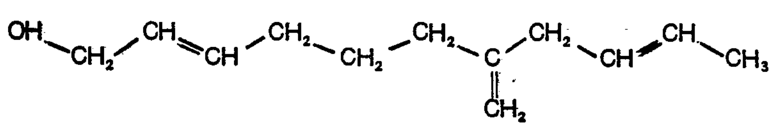

La sélectivité en méthylène-7 undécadiène 2,9 ol-1 est de 67% pour un taux de transformation de 77%.

Exemple 6

Préparation de l'acétoxy-1 méthylène-7 undécadiène-2,9 à partir de butadiène-1,3 et d'acétoxy-1 octadiène-2,7

$$\text{(rapport atomique } \frac{\text{phosphore trivalent}}{\text{rhodium}} = 4)$$

On opère comme dans l'exemple 4 mais on utilise 0,210 g de triphénylphosphine (0,8 millimole) et 0,8 g d'éthanol.

On obtient:

— 0,11 g du composé iso

— 0,8 g du composé linéaire

— 4,72 g d'acétoxy-1 méthylène-7 undécadiène 2,9

La sélectivité en acétoxy-1 méthylène-7 undécadiène-2,9 est de 84% pour un taux de transformation de l'acétoxy-1 octadiène-2,7 de 38%.

Exemple 7

Préparation de l'acétoxy-1 méthylène-7 undécadiène-2,9 à partir de butadiène-1,3 et d'acétoxy-1 octadiène-2,7

$$\left(\text{rapport atomique } \frac{\text{phosphore trivalent}}{\text{rhodium}} = 6\right)$$

On opère comme dans l'exemple 4 mais on utilise 0,314 g de triphénylphosphine (1,2 millimole) et 0,8 g d'éthanol.

On obtient:

— 0,02 g de composé iso

— 0,30 g de composé linéaire

2,23 g d'acétoxy-1 méthylène-7 undécadiène-2,9.

La sélectivité en acétoxy-1 méthylène-7 undécadiène-2,9 est de 87% pour un taux de transformation de l'acétoxy-1 octadiène-2,7 de 18%.

Il est à remarquer qu'un rapport atomique

$$\frac{\text{phosphore trivalent}}{\text{rhodium}}$$

supérieur à 3 n'augmente pas la sélectivité de la formation de l'acétoxy-1 méthylène-7 undécadiène-2,9 et diminue le taux de transformation de l'acétoxy-1 octadiène-2,7.

Exemple 8

Préparation du méthoxy-1 méthylène-7 undécadiène-2,9 à partir de méthoxy-1 octadiène-2,7 et de butadiène-1,3

$$\left(\text{rapport atomique } \frac{\text{phosphore trivalent}}{\text{rhodium}} = 3\right).$$

On introduit dans un autoclave en acier inoxydable de 125 cm3, 0,056 g de $Rh\ Cl_3, 4H_2O$ (0,2 millimole), 0,157 g de triphénylphosphine (0,6 millimole), 0,8 g d'éthanol, 20 g de méthoxy-1 octadiène-2,7 et 8,8 g de butadiène 1,3. On chauffe sous agitation à 120°C pendant 6 heures et demie. On élimine le butadiène n'ayant pas réagi et on obtient 29,2 g d'un liquide orangé.

L'analyse chromatographique en phase gazeuse et l'analyse infra rouge, RMN $^1H$ et spectrographique de masse montrent que le mélange réactionnel obtenu est constitué par:

— 1,1 g de produit iso

— 1,5 g de produit linéaire

— 11,37 g de méthoxy-1 méthylène-7 undécadiène-2,9.

La sélectivité en méthoxy-1 méthylène-7 undécadiène-2,9 est de 82% pour un taux de transformation du méthoxy-1 octadiène-2,7 de 90%.

Exemple 9

Préparation du méthoxy-1 méthylène-7 undécadiène-2,9 à partir de méthoxy-1 octadiène-2,7 et de butadiène-1,3

$$\left(\text{rapport atomique } \frac{\text{phosphore trivalent}}{\text{rhodium}} = 5\right).$$

On opère comme dans l'exemple 8 mais en utilisant 0,262 g de triphénylphosphine (1 millimole).

On obtient:

— 0,21 g de composé iso

— 0,9 g de composé linéaire

— 5,41 g de méthoxy-1 méthylène-7 undécadiène-2,9.

La sélectivité en méthoxy-1 méthylène-7 undécadiène-2,9 est de 83% pour un taux de transformation du méthoxy-1 octadiène-2,7 de 40%.

**Revendications**

1. Composé undécadiène fonctionnalisé comportant un substituant de type méthylène répondant à la formule générale:

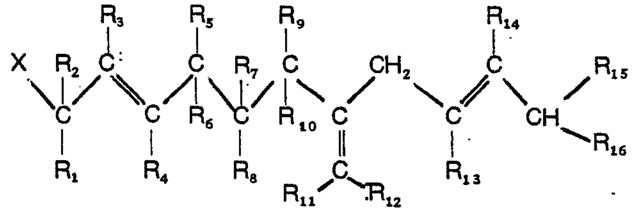

(I)

dans laquelle

X représente un groupement choisi parmi les groupements acyloxy, aryloxy, alkoxy et hydroxy.

$R_1$, $R_2$, $R_4$, $R_7$, $R_8$, $R_{13}$, $R_{14}$, $R_{15}$ et $R_{16}$ identiques ou différents représentent chacun un atome d'hydrogène ou un radical alkyle ayant de 1 à 3 atomes de carbone.

$R_3$, $R_5$ et $R_6$ représentent un atome d'hydrogène ou un radical alkyle ayant de 1 à 3 atomes de carbone au moins l'un d'entre eux étant un atome d'hydrogène.

$R_9$, $R_{10}$, $R_{11}$ et $R_{12}$ représentent un atome d'hydrogène ou un radical alkyle ayant de 1 à 3 atomes de carbone, au moins l'un d'entre eux étant un atome d'hydrogène.

2. Composé selon la revendication 1 caractérisé en ce que

$R_1$, $R_2$, $R_4$, $R_7$, $R_8$, $R_{13}$, $R_{14}$, $R_{15}$ et $R_{18}$ identiques ou différents représentent chacun un atome d'hydrogène ou un radical méthyle

$R_3$, $R_5$ et $R_6$ représentent un atome d'hydrogène ou un radical méthyle, au moins l'un d'entre eux étant un atome d'hydrogène

$R_9$, $R_{10}$, $R_{11}$ et $R_{12}$ représentent un atome d'hydrogène ou un radical méthyle, au moins l'un d'entre eux étant un atome d'hydrogène.

3. Composé selon la revendication 2 caractérisé en ce que $R_1$, $R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$ et $R_{16}$ représentent des atomes d'hydrogène.

4. Composé selon la revendication 1 caractérisé en ce que X représente le groupement acétoxy.

5. Composé selon les revendications 3 et 4 caractérisé en ce qu'il répond à la formule

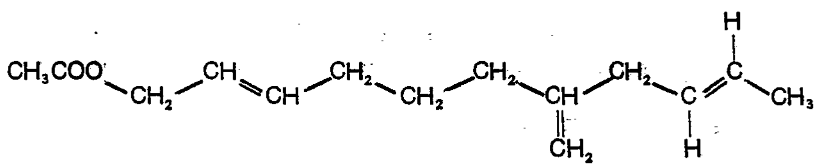

6. Procédé de préparation d'un composé selon la revendication 1 caractérisé en ce que l'on fait réagir un dimère fonctionnalisé de diène conjugé de formule

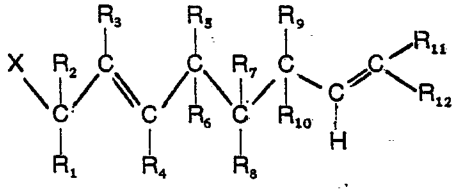

(II)

dans laquelle

X représente un groupement choisi parmi les groupements acyloxy, aryloxy, alkoxy et hydroxy.

$R_1$, $R_2$, $R_4$, $R_7$ et $R_8$ identiques ou différents représentent chacun un atome d'hydrogène ou un radical alkyle ayant de 1 à 3 atomes de carbone

$R_3$, $R_5$ et $R_6$ représentent un atome d'hydrogène ou un radical alkyle ayant de 1 à 3 atomes de carbone, au moins l'un d'entre eux étant un atome d'hydrogène

$R_9$, $R_{10}$, $R_{11}$ et $R_{12}$ représentent un atome d'hydrogène ou un radical alkyle ayant de 1 à 3 atomes de carbone, au moins l'un d'entre eux étant un atome d'hydrogène avec un diène conjugué de formule générale

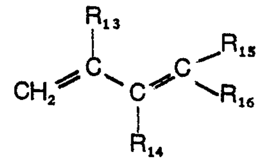

(III)

dans laquelle $R_{13}$, $R_{14}$, $R_{15}$ et $R_{16}$ identiques ou différents représentent chacun un atome d'hydrogène ou un radical alkyle ayant de 1 à 3 atomes de carbone
en présence d'un système catalytique constitué d'une part par un composé du rhodium et d'autre part par un composé trivalent du phosphore, le rapport atomique

$$\frac{\text{phosphore trivalent}}{\text{rhodium}}$$

étant supérieur ou égal à 1,5.

7. Procédé selon la revendication 6 caractérisé en ce que le rapport atomique

$$\frac{\text{phosphore trivalent}}{\text{rhodium}}$$

est compris entre 1,5 et 10.

8. Procédé selon la revendication 7 caractérisé en ce que le rapport atomique

$$\frac{\text{phosphore trivalent}}{\text{rhodium}}$$

est compris entre 2 et 6.

9. Procédé selon la revendication 6 caractérisé en ce que le composé du rhodium est un sel d'acide inorganique de rhodium.

10. Procédé selon la revendication 9 caractérisé en ce que le composé du rhodium est $RhCl_3$.

11. Procédé selon la revendication 6 caractérisé en ce que le composé trivalent du phosphore est choisi parmi les aryl et alkyl phosphines.

12. Procédé selon la revendication 11 caractérisé en ce que le composé trivalent du phosphore est la triphenylphosphine ou la bis (diphenylphosphino)-1,2 éthylène.

13. Procédé selon la revendication 6 caractérisé en ce que le système catalytique est constitué par $RhCl_3$ et la triphenylphosphine.

14. Procédé selon la revendication 6 caractérisé en ce que dans le composé de formule II

$R_1$, $R_2$, $R_4$, $R_7$ et $R_8$ identiques ou différents représentent chacun un atome d'hydrogène ou un radical méthyle

$R_3$, $R_5$ et $R_6$ représentent un atome d'hydrogène ou un radical méthyle, au moins l'un d'entre eux étant un atome d'hydrogène

$R_9$, $R_{10}$, $R_{11}$ et $R_{12}$ représentent un atome d'hydrogène ou un radical méthyle, au moins l'un d'entre eux étant un atome d'hydrogène.

15. Procédé selon la revendication 6 caractérisé en ce que dans le composé de formule III, $R_{13}$, $R_{14}$, $R_{15}$ et $R_{16}$ identiques ou différents représentent chacun un atome d'hydrogène ou un radical méthyle.

16. Procédé selon la revendication 6 caractérisé en ce que le composé de formule II est

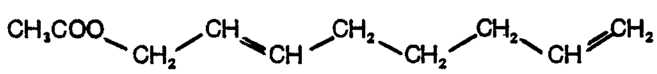

et en ce que le composé de formule III est le butadiène-1,3.

17. Procédé selon l'une quelcc ue des revendications précédentes caractérisé en ce que l'on opère à une température comprise e 90°C et 150°C.

**Claims**

1. A methylenically-substituted undecadiene compound having the structural formula:

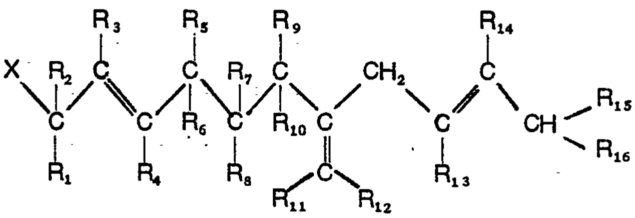

wherein:
— X is a group selected from acyloxy, aryloxy, alkoxy and hydroxy groups,
— $R_1$, $R_2$, $R_4$, $R_7$, $R_8$, $R_{13}$, $R_{14}$, $R_{15}$ and $R_{16}$ identical or different are hydrogen or alkyl groups having from 1 to 3 carbon atoms,
— $R_3$, $R_5$ and $R_6$ are hydrogen or alkyl groups having from 1 to 3 carbon atoms, at least one of them being hydrogen,
— $R_9$, $R_{10}$, $R_{11}$ and $R_{12}$ are hydrogen or alkyl groups having from 1 to 3 carbon atoms; at least one of them being hydrogen.

2. Compound according to claim 1 characterized by the fact that:
— $R_1$, $R_2$, $R_4$, $R_7$, $R_8$, $R_{13}$, $R_{14}$, $R_{15}$ and $R_{16}$ identical or different are hydrogen or methyl groups
— $R_3$, $R_5$ and $R_6$ are hydrogen or methyl groups, at least one of them being hydrogen,
— $R_9$, $R_{10}$, $R_{11}$ and $R_{12}$ are hydrogen or methyl groups, at least one of them being hydrogen.

3. Compound according to claim 2 characterized by the fact that:
— $R_1$, $R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$ and $R_{16}$ are hydrogen.

4. The compound according to claim 1 characterized by the fact that X is an acetoxy group.

5. The compound according to claims 3 and 4 characterized by the structural formula:

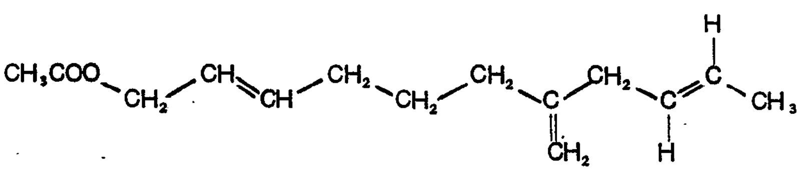

6. A process for the preparation of the compound of claim 1 characterized by the fact that a substituted diene dimer having the structural formula:

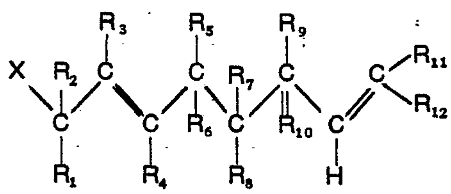

wherein:
— X is a group selected from acyloxy, aryloxy, alkoxy and hydroxy groups,
— $R_1$, $R_2$, $R_4$, $R_7$ and $R_8$ identical or different are hydrogen or alkyl groups having from 1 to 3 carbon atoms, at least one of them being hydrogen,
— $R_9$, $R_{10}$, $R_{11}$ and $R_{12}$ are hydrogen or alkyl groups having from 1 to 3 carbon atoms, at least one of them being hydrogen,

is reacted with a diene having the structural formula:

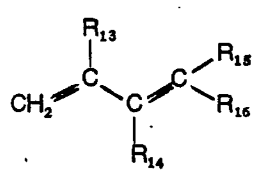

wherein $R_{13}$, $R_{14}$, $R_{15}$ and $R_{16}$ identical or different are hydrogen or alkyl groups having from 1 to 3 carbon atoms, in the presence of a catalytic system constituted by a rhodium compound and a trivalent phosphorous compound, the atomic ratio of trivalent phosphorous to rhodium being greater or equal to 1,5.

7. Process according to claim 6 characterized by the fact that the atomic ratio of trivalent phosphorous to rhodium is in the range of 1,5 to about 10.

8. Process according to claim 7 characterized by the fact that the atomic ratio of trivalent phosphorous to rhodium is in the range of 2 to 6.

9. Process according to claim 6 characterized by the fact that the rhodium compound is an inorganic acid salt of rhodium.

10. Process according to claim 9 characterized by the fact that the rhodium compound is $RhCl_3$.

11. Process according to claim 6 characterized by the fact that the trivalent phosphorous compound is selected from aryl and alkyl phosphines.

12. Process according to claim 11 characterized by the fact that the trivalent phosphorus compound is triphenyl phosphine or bis-1,2-(diphenylphosphino)ethylene.

13. Process according to claim 6 characterized by the fact that the catalytic system is constituted by $RhCl_3$ and triphenylphosphine.

14. Process according to claim 6 characterized by the fact that in the formula of compound II:
— $R_1$, $R_2$, $R_4$, $R_7$ and $R_8$ identical or different are hydrogen atom or methyl groups,
— $R_3$, $R_5$ and $R_6$ are hydrogen or methyl groups, at least one of them being hydrogen,
— $R_9$, $R_{10}$, $R_{11}$ and $R_{12}$ are hydrogen or methyl groups, at least one of them being hydrogen.

15. Process according to claim 6 characterized by the fact that in the formula of compound III, $R_{13}$, $R_{14}$, $R_{15}$ and $R_{16}$ identical or different are hydrogen, or methyl groups.

16. Process according to claim 6 characterized by the fact that compound of formula II has the structural formula:

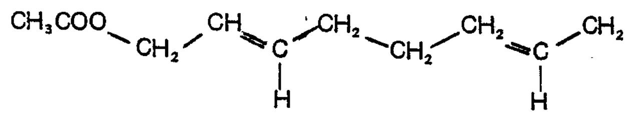

and in that the compound of formula III is 1,3-butadiene.

17. Process according to any one of the preceding claims characterized by the fact that the process is operated in the range of 90°C to 150°C.

**Patentansprüche**

1. Funktionalisierte Undecadien-Verbindung mit einem Substituenten vom Methylen-Typ, der allgemeinen Formel

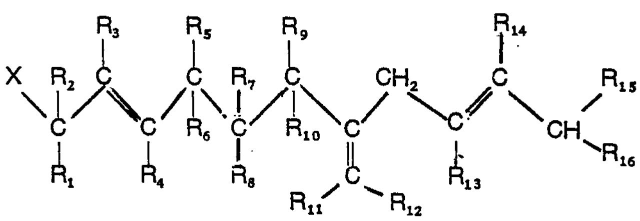

(I)

in der
X eine unter Acyloxy-, Aryloxy-, Alkoxy- und Hydroxygruppen ausgewählte Gruppe bedeutet,
$R_1$, $R_2$, $R_4$, $R_7$, $R_8$, $R_{13}$, $R_{14}$, $R_{15}$ und $R_{16}$ gleich oder unterschiedlich sind und jeweils ein Wasserstoffatom oder einen Alkylrest mit 1 bis 3 Kohlenstoffatomen bedeuten.
$R_3$, $R_5$ und $R_6$ ein Wasseratom oder einen Alkylrest mit 1 bis 3 Kohlenstoffatomen bedeuten, wobei wenigstens einer der Reste ein Wasserstoffatom ist,
$R_9$, $R_{10}$ und $R_{12}$ ein Wasserstoffatom oder einen Alkylrest mit 1 bis 3 Kohlenstoffatomen bedeuten, wobei wenigstens einer der Reste ein Wasserstoffatom ist.

2. Verbindung nach Anspruch 1, dadurch gekennzeichnet, daß die Reste $R_1$, $R_2$, $R_4$, $R_7$, $R_8$, $R_{13}$, $R_{14}$, $R_{15}$ und $R_{16}$ gleich oder unterschiedlich ein Wasserstoffatom oder einen Methylrest bedeuten.
$R_3$, $R_5$ und $R_6$ ein Wasserstoffatom oder einen Methylrest bedeuten, wobei wenigstens einer dieser Reste ein Wasserstoffatom ist,
$R_9$, $R_{10}$, $R_{11}$ und $R_{12}$ ein Wasserstoffatom oder einen Methylrest bedeuten, wobei wenigstens einer dieser Reste ein Wasserstoffatom ist.

3. Verbindung nach Anspruch 2, dadurch gekennzeichnet, daß $R_1$, $R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$ und $R_{16}$ Wasserstoffatome bedeuten.

4. Verbindung nach Anspruch 1, dadurch gekennzeichnet, daß X die Acetoxygruppe bedeutet.

5. Verbindung nach den Ansprüchen 3 und 3, dadurch gekennzeichnet, daß sie der folgenden Formel entspricht:

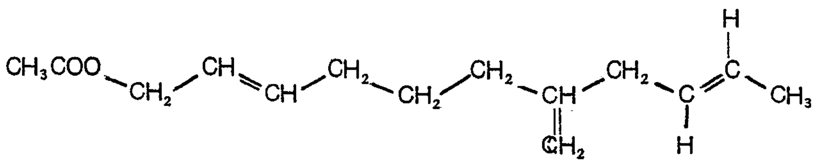

6. Verfahren zur Herstellung einer Verbindung nach Anspruch 1, dadurch gekennzeichnet, daß man ein funktionalisiertes Dimeres eines konjugierten Diens der Formel

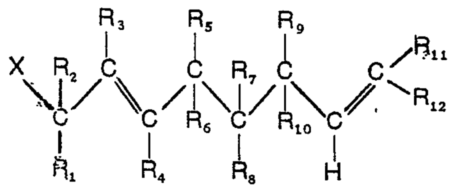

(II)

in der X eine unter Alkoxy-, Aryloxy-, Alkoxy- und Hydorxygruppen ausgewählte Gruppe bedeutet, $R_1$, $R_2$, $R_4$, $R_7$ und $R_8$ gleich oder unterschiedlich jeweils ein Wasserstoffatom oder einen Alkylrest mit 1 bis 3 Kohlenstoffatomen bedeuten,
$R_3$, $R_5$ und $R_6$ ein Wasserstoffatom oder einen Alkylrest mit 1 bis 3 Kohlenstoffatomen bedeuten, wobei wenigstens einer dieser Reste ein Wasserstoffatom ist,
$R_9$, $R_{10}$, $R_{11}$ und $R_{12}$ ein Wasserstoffatom oder einen Alkylrest mit 1 bis 3 Kohlenstoffatomen bedeuten, wobei wenigstens einer der Reste ein Wasserstoffatom ist,
mit einem konjugierten Dien der allgemeinen Formel

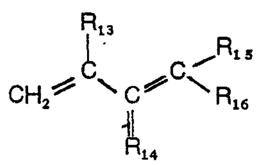

(III)

in der $R_{13}$, $R_{14}$, $R_{15}$ und $R_{16}$ gleich oder unterschiedlich jeweils ein Wasserstoffatom oder einen Alkylrest mit 1 bis 3 Kohlenstoffatomen bedeuten, in Gegenwart eines Katalysatorsystems umsetzt, das einerseits aus einer Rhodiumverbindung und andererseits aus einer dreiwertigen Phosphorverbindung besteht, wobei das Atomverhältnis des dreiwertigen Phosphors zum Rhodium größer oder gleich 1,5 ist.

7. Verfahren nach Anspruch 6, dadurch gekennzeichnet, daß das atomare Verhältnis des dreiwertigen Phosphors zum Rhodium zwischen 1,5 und 10 liegt.

8. Verfahren nach Anspruch 7, dadurch gekennzeichnet, daß das atomare Verhältnis von dreiwertigen Phosphor zu Rhodium zwischen 2 und 6 liegt.

9. Verfahren nach Anspruch 6, dadurch gekennzeichnet, daß die Rhodiumverbindung ein Salz einer anorganischen Säure des Rhodiums ist.

10. Verfahren nach Anspruch 9, dadurch gekennzeichnet, daß die Rhodiumverbindung $RhCl_3$ ist.

11. Verfahren nach Anspruch 6, dadurch gekennzeichnet, daß die dreiwertige Phosphorverbindung unter den Aryl- und Alkylphosphinen ausgewählt ist.

12. Verfahren nach Anspruch 11, dadurch gekennzeichnet, daß die dreiwertige Phosphorverbindung Triphenylphosphin oder Bis-1,2-(diphenylphosphino)-äthylen ist.

13. Verfahren nach Anspruch 6, dadurch gekennzeichnet, daß das Katalysatorsystem aus $RhCl_3$ und Triphenylphosphin besteht.

14. Verfahren nach Anspruch 6, dadurch gekennzeichnet, daß in der Verbindung der Formel II $R_1$, $R_2$, $R_4$, $R_7$, $R_8$ gleich oder unterschiedlich jeweils ein Wasserstoffatom oder einen Methylrest bedeuten,
$R_3$, $R_5$, $R_6$ ein Wasserstoffatom oder einen Methylrest bedeuten, wobei wenigstens einer dieser Reste ein Wasserstoffatom ist,
$R_9$, $R_{10}$, $R_{11}$ und $R_{12}$ ein Wasserstoffatom oder einen Methylrest bedeuten, wobei wenigstens einer dieser Reste ein Wasserstoffatom ist.

15. Verfahren nach Anspruch 6, dadurch gekennzeichnet, daß in der Verbindung der Formel III $R_{13}$, $R_{14}$, $R_{15}$ und $R_{16}$ gleich oder verschieden jeweils ein Wasserstoffatom oder einen Methylrest bedeuten.

16. Verfahren nach Anspruch 6, dadurch gekennzeichnet, daß die Verbindung der Formel II

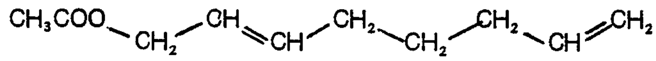

ist und daß die Verbindung der Formel III 1,3-Butadien ist.

17. Verfahren nach irgendeinem der vorangehenden Ansprüche, dadurch gekennzeichnet, daß man bei einer Temperatur zwischen 90°C und 150°C arbeitet.